# EUROPEAN PATENT APPLICATION

(11) **EP 3 785 722 A1**
(43) Date of publication of application: **03.03.2021**
(21) Application number: 19792672.8
(22) Date of filing: 04.01.2019
(51) Int. Cl.: A61K 35/768, A61P 35/00

(54) **PHARMACEUTICAL KIT AND USES THEREOF**

(30) Priority: 24.04.2018 BR 102018008194
(71) Applicant: Universidade De São Paulo, 05508-220 São Paulo - SP (BR)
(72) Inventor: KEITH OKAMOTO, Oswaldo, 06710-750 Cotia (BR); ZATZ, Mayana, 05469-030 São Paulo (BR); KAID DÁVILA, Carolini, 05586-060 São Paulo (BR); DA SILVEIRA GOULART GUIMARÃES, Ernesto, 05410-002 SÃO PAULO (BR); CARLOS DE CAIRES JÚNIOR, Luiz, 05083-120 São Paulo (BR)
(74) Representative: Clarke Modet & Co.
(86) International application number: PCT/BR2019/050002
(87) International publication number: WO 2019/204888

(57) **Abstract**

The present invention relates to a kit for the destruction of cancer cells with stem cell properties based on the oncolytic action of the Zika virus, associated with the aggressiveness of malignant tumors and an unfavorable clinical prognosis, especially in malignant tumors of the nervous system. Said kit comprises a pharmaceutical composition, sterile glass vials with a rubber seal, and syringes with sterile and disposable needles for injection.

Listagem de sequências

<110>  UNIVERSIDADE DE SÃO PAULO - USP

<120>  KIT FARMACÊUTICO E SEUS USOS

<130>  Células Tronco-Tumorais BR 10 2018008194-2

<160>  10

<170>  PatentIn version 3.5

<210>  1
<211>  23
<212>  RNA
<213>  oncomiRs - Sequência (5′ -> 3′)

<400>  1
acuuaaacgu ggauguacuu gcu                                              23

<210>  2
<211>  23
<212>  RNA
<213>  oncomiRs - Sequência (5′ -> 3′)

<400>  2
aguaagugcu uccauguuuu ggu                                              23

<210>  3
<211>  22
<212>  RNA
<213>  oncomiRs - Sequência (5′ -> 3′)

<400>  3
acuuuaacau ggaagugcuu uc                                               22

<210>  4
<211>  25
<212>  RNA
<213>  oncomiRs - Sequência (5′ -> 3′)

<400>  4
aguaagugcu uccauguuuu aguag                                            25

<210>  5
<211>  24
<212>  RNA
<213>  oncomiRs - Sequência (5′ -> 3′)

<400>  5
gcuuuaacau ggggguaccu gcug                                             24

<210>  6
<211>  26
<212>  RNA
<213>  oncomiRs - Sequência (5′ -> 3′)

<400>  6
aguaagugcu uccauguuuc agugga                                          26


<210>  7
<211>  27
<212>  RNA
<213>  oncomiRs - Sequência (5′ -> 3′)

<400>  7
acuuuaacau ggaggcacuu gcuguga                                         27


<210>  8
<211>  24
<212>  RNA
<213>  oncomiRs - Sequência (5′ -> 3′)

<400>  8
auaagugcuu ccauguuuga gugu                                            24


<210>  9
<211>  22
<212>  RNA
<213>  oncomiRs - Sequência (5′ -> 3′)

<400>  9
acuguugcua auaugcaacu cu                                              22


<210>  10
<211>  22
<212>  RNA
<213>  oncomiRs - Sequência (5′ -> 3′)

<400>  10
aauugcacuu uagcaauggu ga                                              22

## Description

### FIELD OF THE INVENTION

The present invention falls within the field of Biology, more precisely in the areas of Molecular Biology and Human Health, and describes a kit for the destruction of cancer cells with stem cell properties based on the oncolytic action of the Zika virus, comprising a pharmaceutical composition, sterile glass vials with rubber seal and syringes with sterile and disposable needles for injection.

### BACKGROUND OF THE INVENTION

Viruses capable of infecting and killing cancer cells are called oncolytic viruses. Preliminary observations of tumor regression after natural viral infections in patients date from the 1950s. However, a series of properly grounded and structured clinical studies to investigate the effect of oncolytic viruses as a new class of drugs for the treatment of solid tumors has only occurred since the early 2000s. Several types of viruses have been tested in phase I to III clinical studies, including therapies with adenovirus, herpes virus, rubella, and smallpox. In general, these clinical studies have reported low toxicity and minimal adverse effects in patients undergoing these experimental treatments (Patel MR, Kratzke RA. Transl Res. 2013 Apr; 161(4):355 to 364).

A modified adenovirus called H101 was approved for the treatment of nasopharyngeal cancer in 2005 in China, and its use is restricted to this country (Liang M. Curr Cancer Drug Targets. 2017 Nov 29). In 2015, a modified form of Herpes simplex virus type 1 (HSV-1), called T-VEC, was approved in the U.S. by the US Food and Drug Administration (FDA) for the treatment of patients with recurrent melanoma. Treatment with T-VEC for this same condition was subsequently approved by the European Medical Agency (EMA), as well as by the Australian Regulatory Agency (Rehman et al. J Immunother Cancer. 2016 Sep 20; 4:53), making therapy with oncolytic viruses a reality for cancer patients.

This new therapeutic approach is in full development. By 2018, more than 70 clinical studies with different viral platforms are underway to treat aggressive forms of cancer, including pancreatic cancer, melanoma, and gliomas (ClinicalTrials.gov, NIH-USA).

Oncolytic viral therapy employs natural or modified viruses capable of preferentially infecting tumor cells (oncotropism) and destroying them (oncolysis). One of the limitations of this therapeutic modality is that most native viruses can infect both cancer cells and normal cells. For this reason, many oncolytic viruses require genetic manipulation to preferentially infect tumor cells. This is the case with the H101 adenovirus, which has the deleted E1B-55k gene, and the modified HSV-1 herpes virus (T-VEC), which has the artificially mutated c34.5 and a47 genes (Fukuhara et al. Cancer Sci. 2016 Oct; 107(10):1373 to 1379).

Genetic manipulations involving the insertion of new transgenes are also carried out on viruses to increase their power to destroy cancer cells. The modified herpesvirus T-VEC itself has an insertion of an encoding gene of human GM-CSF (human granulocyte-macrophage colony-stimulating factor) to stimulate the patient's immune response against cancer cells.

On the other hand, such manipulation of the viral genome to make them safer and increase their antitumor activity is relatively complex and can have undesirable or uncontrollable effects regarding the injection of these viruses in patients.

Another limitation of oncolytic therapy is the restricted ability to generate high titers of viruses with adequate quality to be used clinically. That is the need for high amounts of viruses to obtain desirable oncotropic and oncolytic effects. Besides, current products are based on common viruses such as herpes and rubella, which have reduced efficacy because of the presence of pre-existing antibodies in patients. About 95% of the Western population, for example, has neutralizing antibodies against rubella virus due to vaccination and/or the previous infection.

In this respect, the present invention stands out because it contains in its composition a small and defined amount of virus, for which an effective and specific oncolytic action against cancer cells of the central nervous system (CNS) and low infectivity in other cell types has been determined. The invention also incorporates an injection system that allows direct access of the oncolytic virus to the CNS, minimizing its destruction by the possible presence of neutralizing antibodies that have access to the CNS normally restricted by the blood-brain barrier.

### STATE OF THE ART

The document entitled "ZIKA VIRUS INFECTION INDUCES SYNTHESIS OF DIGOXIN IN GLIOBLASTOMA CELLS" assesses the oncolytic effects of a Zika virus infection on human glioblastoma cells, using endogenous digoxin as a biomarker for the Zika virus/glioblastoma cell line, associated with cytopathic effects induced by a viral infection. However, this document presents some differences concerning the present invention, such as the fact that the description mentions only cytopathic effects *in vitro,* it does not determine cell death; the fact of not showing an oncolytic effect; the fact that the tumor studied is a glioblastoma, different from the group of tumors related to the present invention, where oncolytic effects have been demonstrated *in vitro* and *in vivo* in embryonic tumors of the Central Nervous System, compared to the three tumors with greater occurrence in the population (breast tumor, prostate tumor, and colorectal tumor).

The document entitled "ZIKA VIRUS INFECTS NEURAL PROGENITORS IN THE ADULT MOUSE BRAIN AND ALTERS PROLIFERATION" studies the effects of the Zika virus on neuronal stem cells in both adults and fetuses, which can lead to cell death and reduced proliferation. However, this document only addresses effects on normal brain cells to understand the microcephaly-related effects of the Zika virus and does not refer to tumor cells. In the same way that a virus can infect a certain type of normal cell, but not necessarily other distinct types of normal cells (which is verified for Zika), the fact that Zika infects a normal stem cell does not mean that it also can infect a cancer stem cell.

The document entitled "ONCOLYTIC VIRUSES IN CANCER THERAPY" reviews virotherapy, showing that under certain circumstances, viruses can attack and destroy cancer cells in cancer patients. The document entitled "RECENT PROGRESS IN THE BATTLE BETWEEN ONCOLYTIC VIRUSES AND TUMORS" surveys the stage of development of anticancer therapy using oncolytic viruses. The document entitled "ONCOLYTIC VIRUS" proposes the use of a virus for the manufacture of a drug to reduce the viability of a cancer cell in an individual. So, as can be seen, these documents are examples of the use of certain viruses to destroy tumor cells. However, none of them specifically mention Zika or the group of tumor cells evaluated in the present invention. It is a fact that numerous documents are pointing to the use of monoclonal antibodies for the destruction of tumor cells, but these do not confer sufficient state of the art to jeopardize the patentability requirements of new antibodies specific for this purpose. Similarly, documents on oncolytic viruses do not give precedence to new products on the same theme, since it is not possible to predict the behavior of the microorganism from other established conditions.

Thus, unexpectedly, the kit proposed in the present invention results in the effective destruction of human cancer stem cells, due to a significant increase in cell death by necrosis. Additionally, it also demonstrates the capacity for complete eradication of tumors and metastases in the nervous system of experimental animals, an effect of superior potency, compared to existing methods. In fact, in many types of cancer, the presence of cancer stem cells is associated with a more unfavorable clinical prognosis, due to its high efficiency in initiating the development of tumors, restoring tumor growth after conventional treatments, and spreading metastases from a distance.

### SUMMARY OF THE INVENTION

The purpose of the present invention is to propose a kit for the destruction of cancer cells with stem cell properties based on the oncolytic action of the Zika virus, comprising a pharmaceutical composition, sterile glass vials with rubber seal, and sterile and disposable needle syringes for injection.

### BRIEF DESCRIPTION OF THE FIGURES

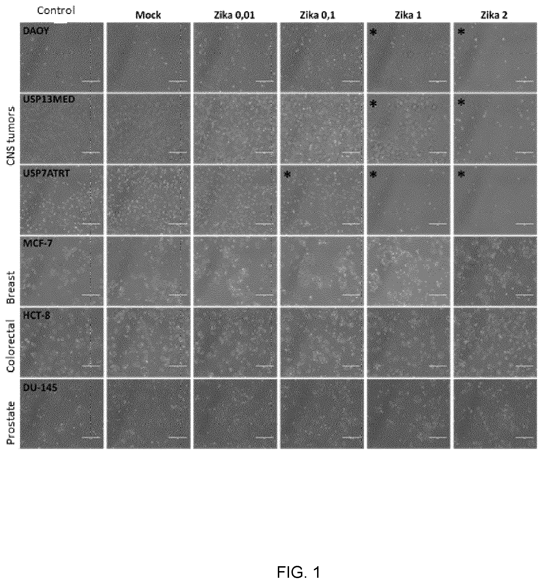

FIG. 1 shows the oncolytic effect of different amounts of the Zika virus (MOI 0.01; 0.1; 1 or 2) on central nervous system tumor cells (cell lines DAOY, USP13-MED and USP7-ATRT), breast tumor (cell line MCF-7), colorectal tumor (cell line HCT-8) and prostate tumor (DU145) * dead cells.

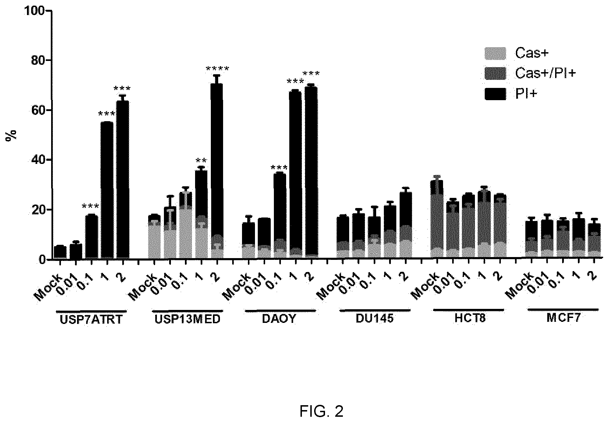

FIG. 2 graphically shows the specific induction of cell death by necrosis after exposure of central nervous system tumor cells (cell lines DAOY, USP13-MED, and USP7-ATRT) to different amounts of the Zika virus (MOI 0.01; 0.1; 1 or 2). Under the same experimental conditions, breast tumor cells (cell line MCF-7), colorectal tumor (cell line HCT-8), and prostate tumor (DU145) did not suffer death. Statistical significance: *** p < 0.005; **** p < 0.001. Black Bars = % of cells killed by necrosis (PI+); Red Bars = % of cells killed in late apoptosis (Cas+Pl+); Gray Bars = % of cells killed in initial apoptosis.

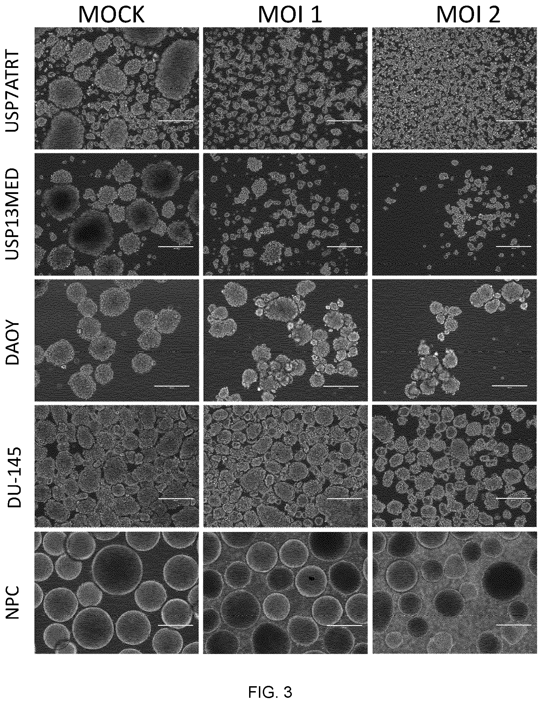

FIG. 3 shows the oncolytic effect of different amounts of the Zika virus (MOI 1 or 2) on tumorspheres composed of stem cells from central nervous system tumors (cell lines DAOY, USP13-MED and USP7-ATRT), prostate tumor (DU145), and normal human neural stem cells (NPC) * dead cells.

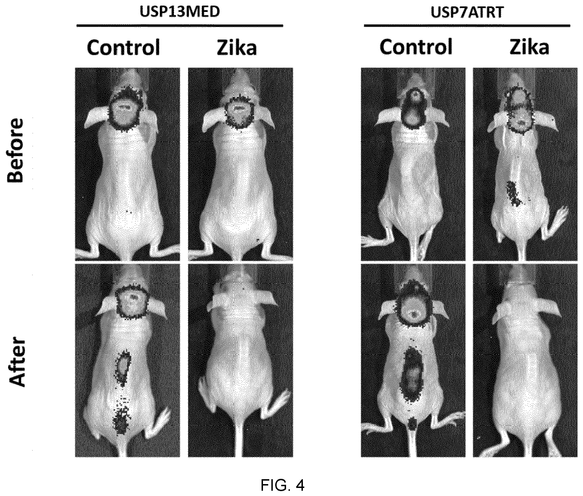

FIG. 4 shows the effect of the Zika virus on the development of tumors of the central nervous system and their metastases in the spinal cord. Control = animals that received intracerebroventricular injection (i.c.v.) of vehicle; Zika = animals that received i.c.v. of Zika virus (6 x 10³ pfu). Before = images showing the existence and location of tumors before injection; After = images of the same animals, two weeks post-injection.

### DETAILED DESCRIPTION OF THE INVENTION

The present invention relates to a kit for destroying cancer cells with stem cell properties based on viral oncolytic action comprising a pharmaceutical composition, five sterile glass vials with a rubber seal, and five syringes with sterile and disposable needles for injection.

Said pharmaceutical composition comprises a sufficient number of viral particles for the administration of Zika virus in concentrations that can vary from 0.01 to 10 MOI (multiplicity of infection), solubilized in a buffering and sterile agent (pH range between 6 and 8), stabilizing agent, osmolarity regulating agents, immunological adjuvants, oncomiRs inhibitors, and chemotherapeutic agents.

The Zika virus used in the kit can be wild, from different isolates of the virus, or any genetic variant of the virus-containing changes naturally acquired or artificially inserted. Examples of genetic changes include, but are not limited to, changes that promote less deleterious effect in normal cells, greater oncolytic effect, greater specificity to tumor cells, greater ability to resist the defense of the immune system, carrying exogenous genes, among others.

The buffering agent can be potassium phosphate or sodium phosphate (final concentration between 1 to 10 mM), tris(hydroxymethyl)aminomethane (final concentration between 5 to 20 mM), or 4-(2-hydroxyethyl)piperazine-1-ethanesulfonic acid (final concentration between 5 to 20 mM), among others.

Stabilizing agents include, but are not limited to, sucrose (final concentration between 2% to 15%), glycerol (final concentration between 0.1% to 10%), non-ionic surfactant, for example, Pluronic® F-68 (final concentration between 0.0001% to 1%), trehalose (final concentration between 1% to 10%), mannitol (final concentration between 5% to 15%), sorbitol (final concentration between 1% to 10%), arginine (final concentration between 0.1% to 10%), glutamine (final concentration between 0.1% to 10%), polyethylene glycol (final concentration between 1 □M to 200 □M), polyoxyethylene-polyoxypropylene copolymers (final concentration between 1% to 10%), serum albumin (final concentration between 0.5% to 5%), monosodium glutamate 1 □M to 1 mM, or combinations between two or more stabilizing agents.

Osmolarity regulating agents include, but are not limited to, sodium chloride (0.1 to 10%), magnesium chloride (0.1 to 10%), sodium citrate (0.1 to 10%, potassium chloride (0.1 to 10%), calcium chloride (0.1 to 10%), glucose (0.1 to 30%), or combinations between two or more osmolarity regulating agents.

Immune adjuvants include, but are not limited to, aluminum phosphate (0.1 to 10%), potassium phosphate (0.1 to 10%), virosomes (0.1 to 10%), tocopherol (0.1 to 10%), squalene (0.1 to 10%), or combinations of two or more immunological adjuvants.

Also, said pharmaceutical composition may include the addition of oncomiR inhibitors (Table 1), present in a final concentration between 0.1 to 1000 mg/mL.

**Table 1. Examples of oncomiRs and their respective sequences of nitrogenous bases to be inhibited by corresponding antisense sequences.**

| **SEQ ID** | **Sequence (5'** → **3')** |
|---|---|
| 1 | ACUUAAACGUGGAUGUACUUGCU |
| 2 | AGUAAGUGCUUCCAUGUUUUGGU |
| 3 | ACUUUAACAUGGAAGUGCUUUC |
| 4 | AGUAAGUGCUUCCAUGUUUUAGUAG |
| 5 | GCUUUAACAUGGGGGUACCUGCUG |
| 6 | AGUAAGUGCUUCCAUGUUUCAGUGGA |
| 7 | ACUUUAACAUGGAGGCACUUGCUGUGA |
| 8 | AUAAGUGCUUCCAUGUUUGAGUGU |
| 9 | ACUGUUGCUAAUAUGCAACUCU |
| 10 | AAUUGCACUUUAGCAAUGGUGA |

Also, oncomiRs inhibitors may be complexed with low molecular weight cationic substances, such as polyethylenimine (PEI) and its derivatives (at a polymer/inhibitor ratio between 2 and 30) or liposomes (final ratio between 1 to 2 mol%).

Further, the present pharmaceutical composition may contain chemotherapeutic agents in sublethal doses, including, but not limited to, alkylating agents (e.g., platinum derivatives such as carboplatin and cisplatin; nitrosoureas such as carmustine and lomustine; temozolomide; cyclophosphamide; procarbazine), mitotic inhibitors (e.g., taxanes such as taxol and paclitaxel; etoposide; vincristine), antimetabolites (e.g., methotrexate), enzyme inhibitors (e.g., irinotecan, rapamycin), monoclonal antibodies and immunomodulators in general (e.g., anti-VEGF, anti-EGFR, anti-PD1, anti-PDL1, anti-CTLA4, etc.).

The kit includes the following items in its secondary packaging, but is not limited to them: A) 2 to 5 injection syringes (0.1 to 25 mL), adapted to the ideal and safe form of inoculation (needles with an internal diameter of 0.3 to 1 mm and a length of 10 to 200 mm); B) stock container(s) of therapeutic active in its formulation; C) dilution container(s) of the therapeutic active; D) product defrost internal indicator.

The kit must be stored for a specified time between 1 and 12 months, at low temperature (e.g., from -80 °C to 4 °C). Use only one vial per injection. The injection of the product can occur systemically (e.g., intravenous, intra-arterial, intramuscular or subcutaneous) or locally (e.g., intratumoral, in the cavity generated after tumor resection, interstitial, intracerebroventricular, intrathecal), and serves for direct destruction of cancer cells, as a single agent, metastatic tumor cells, cancer cells in both neoadjuvant and adjuvant modes. Besides, the injection of the product aims to increase the awareness of cancer cells to further complementary treatment, with chemotherapy or radiation therapy; destruction of cancer cells in conjunction with conventional treatment, chemotherapy, radiation therapy, immunomodulators, or target therapy; and even greater awareness of cancer cells for further treatment with target therapy or immunomodulators.

### EXAMPLES

The use of limited amounts of viral particles proved to be capable of infecting and inducing necrosis of tumor cells in the nervous system. Tumor cells from cancers other than the nervous system are rarely infected and destroyed (FIG. 1). Such results were obtained in *in vitro* tests (tumor cell cultures grown in 6-well plates according to the ATCC recommendations and infected with ZIKA by incubating for 60 minutes with the virus in different MOIs and monitoring cell death for the following 72 hours) and *in vivo* (NUDE mice that received 1 x 10⁶ human tumor cells by stereotaxy injection methodology and that were subsequently infected with ZIKA [2000 viral particles per animal] in the same tumor injection region).

When infected, cancer cells of the nervous system are destroyed by the process of cell death by necrosis (FIG. 2). Any cells from other types of cancer, other than the nervous system, infected by Zika are not significantly destroyed. The fact that not every cancer cell infected with Zika is destroyed is unexpected and demonstrates, therefore, that this virus has a selective capacity to destroy cancer cells. These results were obtained after infection of NUDE mice with Zika in the same region where human tumor cells were previously injected.

In particular, the present invention demonstrates that the Zika virus is capable of infecting and destroying spheres of nervous system tumors (3D cell culture - spheres forming in DMEM-F12 supplemented with B-27, N2, EGF, and FGF) composed of cells with stem cell properties. The Zika virus is comparatively more efficient at destroying stem cell spheres from nervous system tumors than normal neural stem cell spheres (FIG. 3), again demonstrating an unexpected selective capacity for cell destruction.

In an *in vivo* model (orthotopic and metastatic) of a tumor of the nervous system, the proposed kit demonstrates that it can stabilize tumor growth, slow down the development of the tumor, or even eradicate the tumor. Additionally, this kit demonstrates that it can inhibit the development of metastases in the neuraxis, as well as to eliminate pre-existing tumor metastases (FIG. 4). Such results were obtained after the infection of mice that had previously received tumor cells and that had neuroaxis metastasis or not.

In the case of non-metastatic malignant tumors of the central nervous system, for example, the injection of viral particles is intracranial, in the neuroanatomical region where the primary tumor resides. Such an injection can be combined with other forms of treatment available for these types of tumors. For example, injection of viral particles can be performed during primary tumor resection surgery of the nervous system, immediately after removal of the tumor mass. In the case of metastatic tumors in the neuroaxis, for example, the injection of viral particles can occur intracerebroventricularly, combined with conventional treatment or replacing aggressive treatments such as radiotherapy and chemotherapy.

Thus, the main technological advance of the present invention concerns the development of an effective kit for eliminating a population of human cancer stem cells, which are typically resistant to existing conventional treatments for cancer patients. This kit stands out for its potent oncolytic action of the Zika virus, combined with an infection and selective destruction of cancer stem cells from aggressive malignant tumors of the central nervous system.

Although the invention has been widely described, it is obvious to those skilled in the art that various changes and modifications can be made in order to improve the design without said changes not being covered by the scope of the invention.

## Claims

1. Pharmaceutical kit **characterized by** comprising:
- pharmaceutical composition,
- 2 to 5 sterile glass vials with a rubber seal,
- 2 to 5 injection syringes of 0.1 to 25 mL, with sterile and disposable needles for injection, with an internal diameter of 0.3 to 1 mm and a length of 10 to 200 mm;
- stock container(s) of the pharmaceutical composition;
- product defrost internal indicator.

2. Kit, according to claim 1, **characterized in that** the pharmaceutical composition comprises a sufficient number of viral particles for the administration of Zika virus in concentrations that can vary from 0.01 to 10 MOI (multiplicity of infection), solubilized in buffering and sterile agent (pH range between 6 and 8), stabilizing agent, osmolarity regulating agents, immunological adjuvants, oncomiRs inhibitors, and chemotherapeutic agent.

3. Kit, according to claim 1, **characterized in that** the Zika virus is wild from different isolates of the virus, or any genetic variant of the virus-containing alterations naturally acquired or artificially inserted.

4. Kit, according to claim 3, **characterized in that** genetic alterations are mainly those that promote less deleterious effect in normal cells, greater oncolytic effect, greater specificity to tumor cells, greater capacity to resist the defense of the immune system, and carrying exogenous genes.

5. Kit, according to claim 2, **characterized in that** the buffering agent is mainly potassium phosphate or sodium phosphate in final concentration between 1 to 10 mM, tris(hydroxymethyl)aminomethane in final concentration between 5 to 20 mM, and 4-(2-hydroxyethyl)piperazine-1-ethanesulfonic acid in final concentration between 5 to 20 mM.

6. Kit, according to claim 2, **characterized in that** the stabilizing agent is selected from the group consisting mainly of sucrose in final concentration between 2% to 15%, trehalose in final concentration between 1% to 10%, mannitol in final concentration between 5% to 15%, sorbitol in final concentration between 1% to 10%, polyethylene glycol in final concentration between 1 □M to 200 □M, copolymers of polyoxyethylene-polyoxypropylene in final concentration between 1% to 10%, human serum albumin in final concentration between 0.5% to 5%, or combinations between two or more stabilizing agents.

7. Kit, according to claim 2, **characterized in that** the osmolarity regulating agents are selected from the group consisting mainly of sodium chloride (0.1 to 10%), magnesium chloride (0.1 to 10%), sodium citrate (0.1 to 10%), potassium chloride (0.1 to 10%), calcium chloride (0.1 to 10%), glucose (0.1 to 30%), or combinations between two or more osmolarity regulating agents.

8. Kit, according to claim 2, **characterized in that** the immunological adjuvants are selected from the group consisting mainly of aluminum phosphate (0.1 to 10%), potassium phosphate (0.1 to 10%), virosomes (0.1 to 10%), tocopherol (0.1 to 10%), squalene (0.1 to 10%), or combinations of two or more immunological adjuvants.

9. Kit, according to claim 2, **characterized in that** the oncomiR inhibitors have a final concentration between 0.1 to 1000 mg/mL.

10. Kit, according to claim 2, **characterized in that** the chemotherapeutic agents are selected from the group consisting of sublethal doses, including, but not limited to, platinum-derived alkylating agents such as carboplatin and cisplatin; nitrosoureas such as carmustine and lomustine; temozolomide; cyclophosphamide; procarbazine, mitotic taxane inhibitors such as taxol and paclitaxel; etoposide; vincristine, antimetabolites such as methotrexate, enzyme inhibitors such as irinotecan, rapamycin, monoclonal antibodies and immunomodulators in general such as anti-VEGF, anti-EGFR, anti-PD1, anti-PDL1, anti-CTLA4.

11. Kit, according to any one of claims 1 to 10, **characterized by** being stored for a specified time between 1 and 12 months in low temperature, from -80 °C to 4 °C.

12. Use of the kit as defined in any one of claims 1 to 11, **characterized by** being for the destruction of cancer cells with stem cell properties based on the oncolytic action of the Zika virus.

13. Use, according to claim 12, **characterized by** being for the direct destruction of cancer cells, as a single agent, of metastatic tumor cells, of cancer cells in both neoadjuvant and adjuvant modes.
